(19) Europäisches Patentamt
European Patent Office
Office européen des brevets

(11) Publication number: **0 030 747 A1**

# (12) EUROPEAN PATENT APPLICATION

(21) Application number: **80201054.6**

(51) Int. Cl.³: **G 03 C 7/36**

(22) Date of filing: **07.11.80**

(30) Priority: **06.12.79 GB 7942111**

(43) Date of publication of application: **24.06.81 Bulletin 81/25**

(84) Designated Contracting States: **BE DE FR GB**

(71) Applicant: **AGFA-GEVAERT naamloze vennootschap**
**Septestraat 27**
**B-2510 Mortsel(BE)**

(72) Inventor: **Monbaliu, Marcel Jacob**
**Neuris 9**
**B-2510 Mortsel(BE)**

(72) Inventor: **Van Poucke, Rapael**
**Ringlaan 22**
**B-2600 Berchem(BE)**

## (54) 2-Equivalent yellow-forming colour couplers, their use in the production of photographic colour images, and photographic elements containing such couplers.

(57) Yellow-forming couplers of the acylacetanilide type, which carry on the methylene group between the two carbonyl groups a 1-imidazolyl coupling-off group, viz. 2-arylcarbamoyl-imidazol-1-yl, 2-alkylcarbamoyl-imidazol-1-yl, or 2-aroyl-imidazol-1-yl, more particularly colour couplers corresponding to the formula

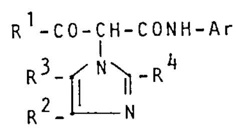

wherein Ar is phenyl or phenyl substituted by 1 to 3 substituents (same or different) such as $C_1$-$C_{18}$ alkyl, $C_1$-$C_{18}$-alkoxy, halogen, acylamido or substituted acylamido, carbamoyl or substituted carbamoyl, sulphamoyl or substituted sulphamoyl, carboxy, alkylsulphonyl, or arylsulphonyl; $R^1$ is $C_1$-$C_{18}$ alkyl, $C_1$-$C_{18}$ alkoxy, alkoxyalkyl, cycloalkyl,heterocyclyl, or aryl, which may be substituted with alkyl, alkoxy, halogen, acyl, acylamido or substituted acylamido, carbamoyl or substituted carbamoyl, sulphamoyl or substituted sulphamoyl, sulphonamido, or carboxy; $R^2$ and $R^3$ are substituents used in colour coupler chemistry, which may be the same or different, or together represent the atoms necessary to complete a 5- or 6- membered unsaturated carbocyclic ring, which may be substituted; $R^4$ is aroyl, arylcarbamoyl, or alkylcarbamoyl.

These yellow-forming colour couplers can be incorporated in the blue-sensitive silver halide emulsion layer of aphotographic multilayer colour element or in a non-light-sensitive colloid layer in water-permeable relationship withthe blue-sensitive emulsion layer.

EP 0 030 747 A1

# 2-Equivalent yellow-forming colour couplers, their use in the production of photographic colour images, and photographic elements containing such couplers.

The present invention relates to novel 2-equivalent yellow-forming colour couplers, to the use thereof in the production of photographic colour images, and to photographic elements containing such colour couplers.

It is known that for the production of a photographic colour image in a light-sensitive silver halide layer, the exposed silver halide is developed to a silver image by means of an aromatic primary amino compound in the presence of a colour coupler, which reacts with the oxidized colour developing agent to form image dye at the areas corresponding to the silver image.

In subtractive three-colour photography use is generally made of a light-sensitive photographic multilayer colour element comprising a red-sensitized silver halide emulsion layer, a green-sensitized silver halide emulsion layer, and a blue-sensitive silver halide emulsion layer, in which material upon colour development, by the use of appropriate colour couplers, a cyan, a magenta, and a yellow dyestuff image are formed respectively.

Usually, cyan-forming colour couplers are phenols or naphthols, magenta-forming colour couplers are pyrazolones, and the yellow-forming colour couplers are compounds comprising a methylene group that carries two adjacent carbonyl groups. The dyes formed by coupling are azomethines, indamines, or indophenols depending on the composition of the colour coupler and of the developing agent.

Many yellow-forming colour couplers have an active methylene group, which during colour development enters into reaction with the oxidized colour developing agent.

During this coupling reaction four equivalents of developable silver halide are needed, which is the reason why these colour couplers are called 4-equivalent colour couplers.

In another group of colour couplers, the so-called 2-equivalent couplers, a hydrogen atom of the active methylene group has been replaced by a coupling-off substituent, which is split off during the coupling reaction. In that case only two equivalents of developable silver halide are needed for the formation of the dyes.

Many coupling-off substituents have been described, e.g. heterocyclic nitrogen-containing substituents as described in the published German Patent Applications DE-OS 2,329,587 filed June 9, 1973 and DE-OS 2,559,190 filed December 30, 1975, both by Agfa-Gevaert A.G. Yet, many of them do not satisfy entirely the photographic requirements.

The above-mentioned German Patent Application DE OS 2,329,587 discloses the use of 2-equivalent yellow-forming couplers having as coupling-off substituent a five-membered unsaturated heterocyclic pyrrole, diazole, triazole, thiazolone-2, or oxazolone-2 nucleus; these nuclei may carry substituents e.g. alkoxycarbonyl substituents. Although these couplers can be readily prepared and have favourable photographic characteristics e.g. high coupling activity, their stability in aqueous acid or alkaline medium is not quite satifactory.

According to the invention there are provided novel colour couplers of the acylacetanilide type,capable of forming yellow azomethine dyes by reaction with an oxidized aromatic primary amino developing agent, said colour couplers carrying on the methylene group between the two carbonyl groups an 1-imidazolyl coupling off group, characterized in that said 1-imidazolyl group is a 2-arylcarbamoyl-imidazol-1-yl group, a 2-alkylcarbamoyl-

imidazol-1-yl group, or a 2-aroyl-imidazol-1-yl group.

More particularly, in accordance with the present invention there are provided novel 2-equivalent yellow-forming colour couplers corresponding to the following general formula :

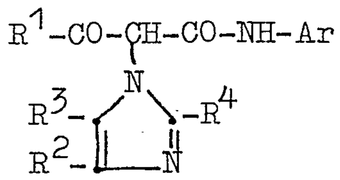

wherein :

Ar represents phenyl or phenyl substituted by 1 to 3 substituents, which may be the same or different and are selected from the group consisting of a branched or unbranched alkyl group having 1 to 18 carbon atoms, an alkoxy group having 1 to 18 carbon atoms e.g. methoxy and n-hexadecyloxy, a halogen atom e.g. chlorine, acylamido or substituted acylamido e.g. (di)alkyl-substituted phenoxyacylamido, carbamoyl or substituted carbamoyl, sulphamoyl or substituted sulphamoyl e.g.N,N-dialkyl-substituted sulphamoyl, carboxy, nitro, alkylsulphonyl, or arylsulphonyl,

$R^1$ represents a branched or unbranched alkyl group having 1 to 18 carbon atoms, wherein in the case of a secondary or tertiary alkyl group the secondary or tertiary carbon atom is preferably linked directly to the carbonyl group e.g. a tert-butyl group, an alkoxy group having 1 to 18 carbon atoms, an alkoxyalkyl group, a cycloalkyl group, a heterocyclic group, an aryl group, or an aryl group substituted with alkyl, alkoxy e.g. methoxyphenyl and n-hexadecyl-oxyphenyl, a halogen atom, acyl, acylamido or substituted acylamido, carbamoyl or substituted carbamoyl, sulphamoyl or substituted sulphamoyl, sulphonamido, or carboxy;

$R^1$ preferably being tert-butyl, phenyl, or substituted phenyl,

$R^2$ and $R^3$ are substituents used in colour coupler chemistry, which may be the same or different, these substituents being hydrogen, alkyl e.g. methyl, alkoxy, alkylthio, aryl or substituted aryl, acylamido or substituted acylamido, carbamoyl or substituted carbamoyl, sulphamoyl or substituted sulphamoyl, sulphoamino, alkoxycarbonyl e.g. ethoxycarbonyl, carboxy, a halogen e.g. a chlorine atom, or cyano; or $R^2$ and $R^3$ together represent the atoms necessary to complete a 5- or 6- membered unsaturated carbocyclic ring e.g. a cyclopentene, cyclohexene, cyclopentadiene, or benzene ring, which may be substituted e.g. with alkyl, alkoxy, alkylthio, aryl or substituted aryl, acylamido or substituted acylamido, carbamoyl or substituted carbamoyl, sulphamoyl or substituted sulphamoyl, sulphoamino, alkoxycarbonyl, carboxy, nitro, a halogen atom, cyano, or trifluoroalkyl; and

$R^4$ represents an aroyl group, an arylcarbamoyl group, or an alkylcarbamoyl group.

The present invention also provides a photographic multilayer colour element comprising three silver halide emulsion layers, which are differently optically sensitized and wherein the novel colour coupler(s) as set forth above is (are) present in a blue-sensitive silver halide emulsion layer or in a non-light-sensitive colloid layer in water-permeable relationship therewith.

The invention also provides the production of a photographic colour image by development of a photographic element containing image-wise exposed silver halide with the aid of a developing agent, which by reduction of the exposed silver halide is converted into its oxidized form and as such forms a yellow azomethine dye by reaction with a novel colour coupler as set forth above.

The yellow-forming couplers of the invention possess in addition to high coupling activity, a high solvolytic stability in acid as well as in alkaline aqueous medium

in contrast with many other 2-equivalent yellow-forming couplers that comprise a heterocyclic coupling-off group and which hydrolyse in acid and/or alkaline medium.

The colour couplers of the invention can be readily prepared by reaction between an appropriately substituted 2-equivalent acylacetanilide colour coupler with chlorine as a group that can be split off and a 2-arylcarbamoyl-imidazole, a 2-alkylcarbamoyl-imidazole, or a 2-aroyl-imidazole.

The 2-arylcarbamoyl-imidazoles and 2-alkylcarbamoyl-imidazoles can be prepared readily and inexpensively according to the method described by E.P. Papadopoulos in Journal of Organic Chemistry, 42, 3925-29 (1977) according to the reaction scheme :

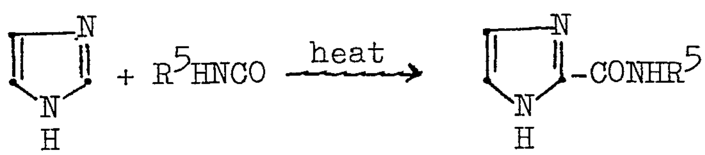


wherein $R^5$ is an alkyl group or an aryl group.

The 2-acyl-imidazoles can be prepared easily and very economically according to the following reaction scheme :

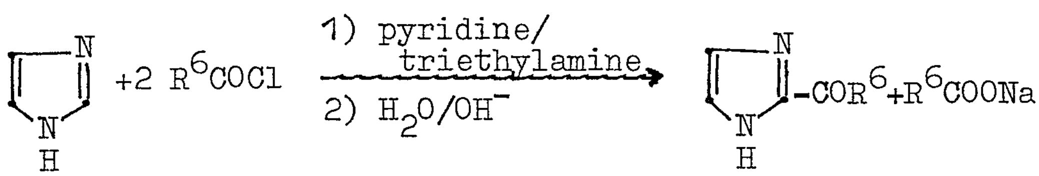


$R^6$ being an aryl group.

This method has been described by L.A.M. Bastiaansen and E.R. Godefroi in Synthesis 676-7 (1978).

Representative examples of novel yellow-forming colour couplers corresponding to the above general formula are given in the following table 1. However, it is to be understood that the invention is not limited to these specifically mentioned colour couplers.

TABLE 1

| | | Melting point °C |
|---|---|---|
| 1. | 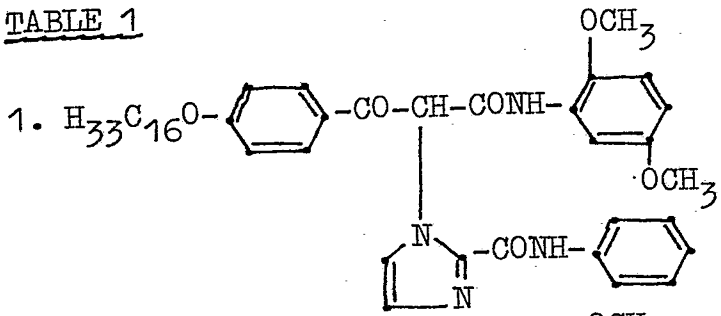 | 101 |
| 2. | 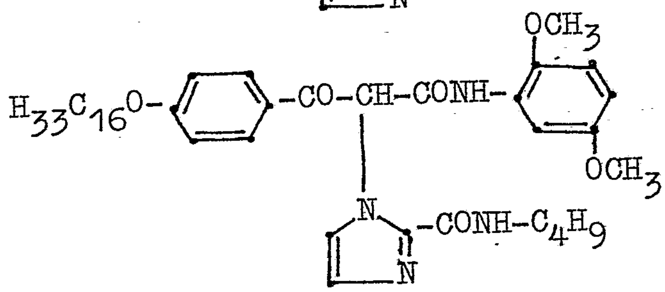 | 118 |
| 3. | 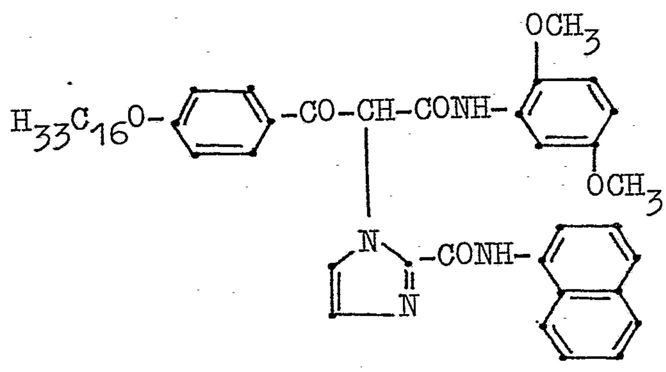 | 82 |
| 4. | 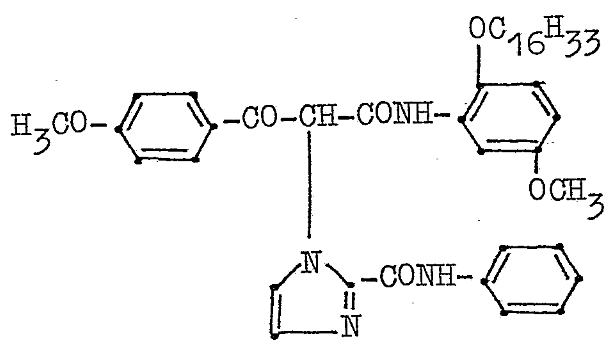 | 70 |
| 5. | 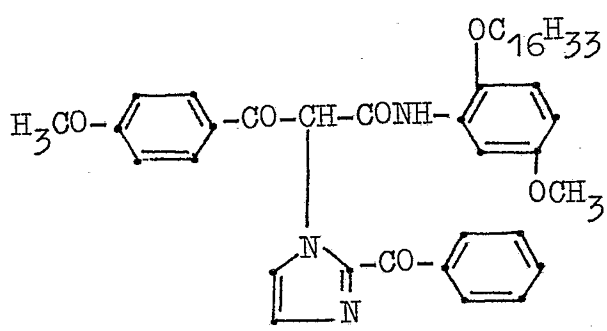 | 88 |

6. 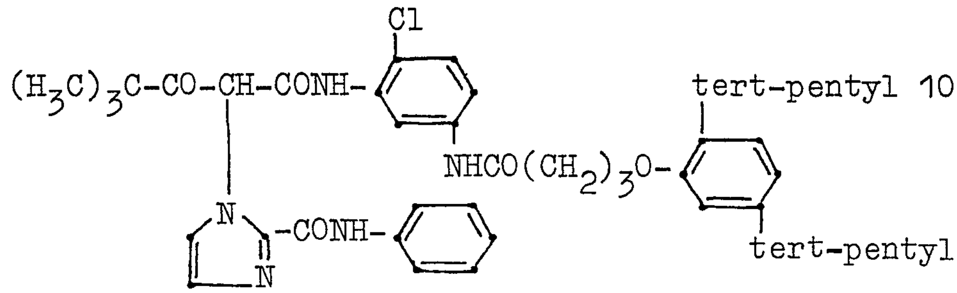 103

7. 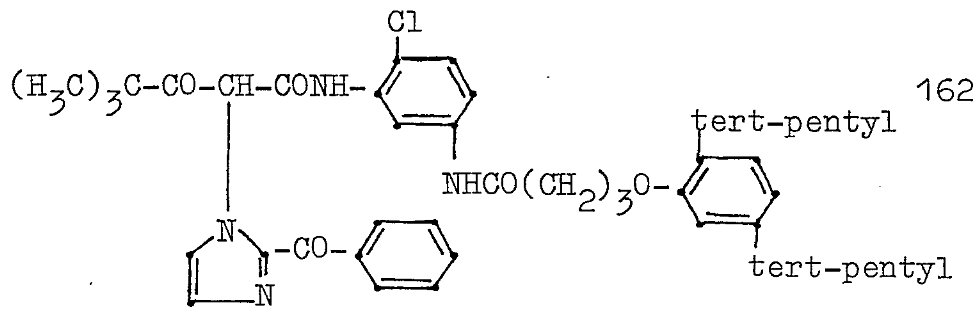 162

8. 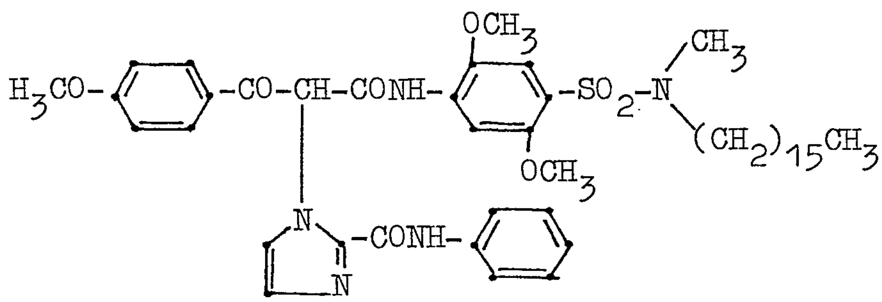 60

9. 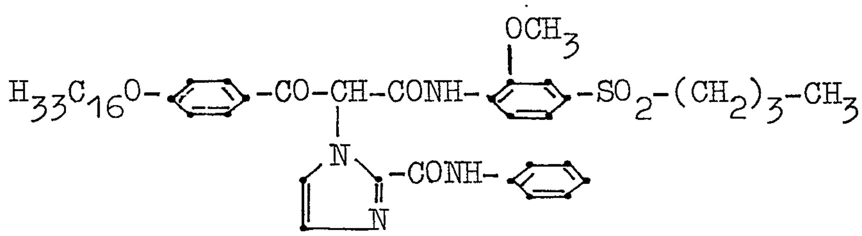 <50

10. 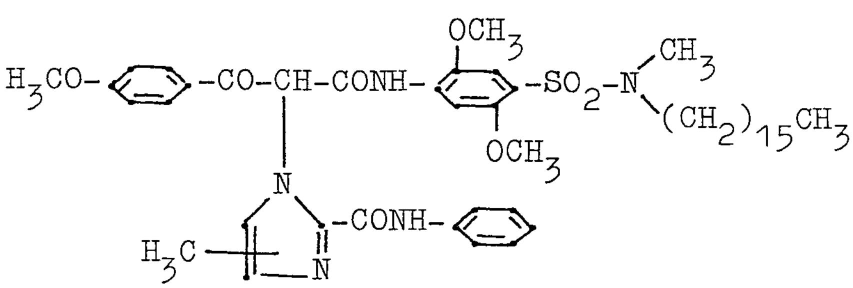 145

11. 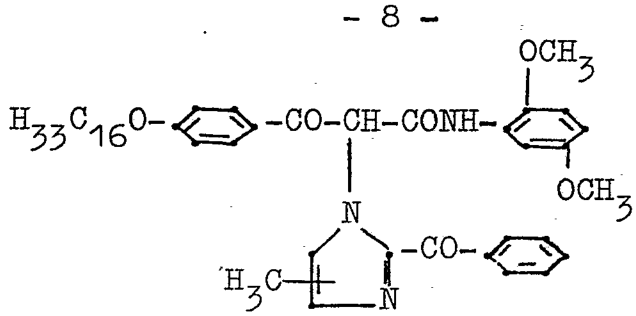 77

12. 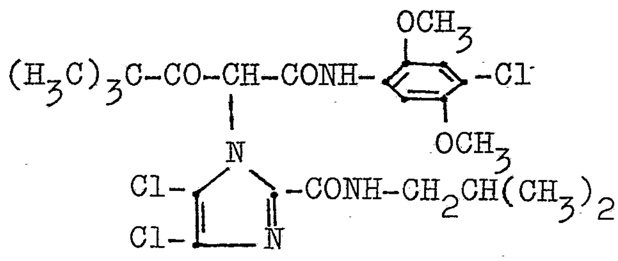 131

Compounds 1, 5, 7, and 10 can be prepared as described in the preparations hereinafter. The preparation of the other compounds of table 1 as well as other compounds corresponding to the general formula but not specifically identified herein can be derived from these preparation examples and will not cause difficulties to those skilled in the art of preparative organic chemistry.

Preparation 1 : compound 1

An amount of 186.8 g (0.5 mole) of α-chloro, α-(4-n-hexadecyloxybenzoyl)-2,5-dimethoxyacetanilide was added to a solution consisting of 93.5 g (0.5 mole) of 2-(phenylcarbamoyl)imidazole, 1500 ml of dimethylformamide, and 125 ml (1 mole) of tetramethylguanidine. The mixture was heated for 1 h at 50°C and poured out in 3.5 l of water and 500 ml of concentrated hydrochloric acid. The product was extracted with dichloromethane. The organic phase was washed until free from acid and the solvent was evaporated. The residual soft mass was recrystallized from a 1 l mixture of acetonitrile and isopropanol (9:1). Yield : 235 g.

Preparation 2 : compound 5

28.68 g (0.05 mole) of α-chloro, α-(4-methoxybenzoyl)-2-n-hexadecyloxy-5-methoxy-acetanilide were added to a solution of 8.6 g (0.05 mole) of 2-benzoyl-imidazole (for the preparation of this compound there can be referred to Synthesis 676-7 (1978) by L.A.M. Bastiaansen and E.R. Godefroi), 150 ml of dimethylformamide, and 12.5 ml of tetramethylguanidine. The mixture was stirred for 8 h at room temperature and then poured out in 1 l of 5 N hydrochloric acid. The product was extracted with dichloromethane. After washing until free from acid and concentration by evaporation of the extract, the oily residue was dissolved in 50 ml of 2-methoxyethanol and then diluted with 250 ml of methanol.
Yield : 24.5 g.

Preparation 3 : compound 7

At room temperature 30.3 g (0.05 mole) of α-chloro, α-pivaloyl-2-chloro-5-[4'-(2,4-di-tert-pentylphenoxy) butyramido]-acetanilide were added to a solution consisting of 8.6 g (0.05 mole) of 2-benzoyl-imidazole, 150 ml of dimethylacetamide, and 14.9 ml (0.1 mole) of 1,8-diazabicyclo (5,4,0)-undec-7-ene. The solution was heated for 1 h at 40°C and poured out in 500 ml of water and 500 ml of 5 N hydrochloric acid. The product was extracted with dichloromethane. The extract was washed until free from acid, dried over magnesium sulphate, and concentrated by evaporation. The residue was recrystallized from a mixture of ethylacetate and ethanol (1:9).
Yield : 31 g.

Preparation 4 : compound 10

27.2 g (0.04 mole) of α-chloro, α-(4-methoxybenzoyl)-2,5-dimethoxy-4-(N-methyl, N-n-hexadecyl)-sulphonamido-acetanilide were added to a solution of 8 g (0.04 mole) of 2-(phenylcarbamoyl)-4- or 5-methylimidazole, 125 ml of dimethylformamide, and 10 ml of tetramethylguanidine. Because of tautomerization the exact position of the methyl group of 2-(phenylcarbamoyl)-4- or 5-methylimidazole cannot be given. The solution was heated for 1 h at 70°C and poured out in 500 ml of water. The pH-value of the solution was adjusted to 3 by means of hydrochloric acid. The product was extracted with dichloromethane and the extract was washed with a sodium hydrogen carbonate solution until free from acid. The extract was dried over magnesium sulphate, and concentrated by evaporation. The oily residue was crystallized from a mixture of 50 ml of ethyl acetate and 200 ml of ethanol. Yield : 19 g.

As mentioned before the yellow-forming couplers of the invention have a high solvolytic stability in acid as well as in alkaline medium. In the following table 2 the solvolytic stability of colour couplers of the invention is compared with that of analogous known compounds, which, however, do not comprise a 2-arylcarbamoyl-substituted imidazole, a 2-alkyl-carbamoyl-substituted imidazole, or a 2-aroyl-substituted imidazole group.

The solvolytic stability of these couplers was determined by subjecting a 0.01 molar solution of a given coupler in a mixture of methanol and a buffer (1:1), after having been heated for 20 h at 50°C, to thin layer chromatography. The solvolytic stability was evaluated from the decrease in intensity of the colour coupler spot and the increase of the spots of other reaction products or degradation products. The buffer can be alkaline or acidic, the alkaline buffer (pH = 8) being prepared from 58.3 ml of a solution of 0.02 mole of salicylic acid in methanol and 41.7 ml of a solution of 0.02 mole of sodium methylate in methanol. The acid buffer (pH = 3) was prepared from 58.8 ml of

a solution of 0.02 mole of p-toluenesulphonic acid and 41.2 ml of a solution of 0.02 mole of sodium methylate in methanol. These buffers have been described by D.D. Perrin and Boyd Demprey in "Buffers for pH and metal ion control" page 91. Chapmann and Hall, London 1974.

TABLE 2

Solvolytic stability of compounds according to the formula :

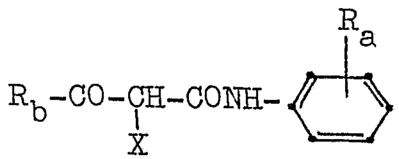

| Colour Coupler* | $R_b$ | $R_a$ | X | pH 3 | pH 8 |
|---|---|---|---|---|---|
| comparison | $H_{33}C_{16}O$- 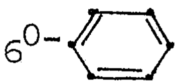 - | 2,5-di-$H_3CO$- | imidazolyl | very instable | very instable |
| comparison | $H_{33}C_{16}O$- 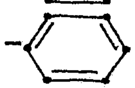 - | 2,5-di-$H_3CO$- | theophyllinyl | very instable | very instable |
| comparison | $H_{33}C_{16}O$- 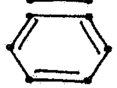 - | 2,5-di-$H_3CO$- | 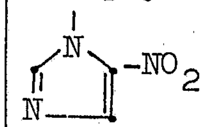 | very instable | very instable |
| comparison | $H_{33}C_{16}O$- 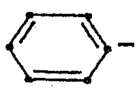 - | 2,5-di-$H_3CO$- | 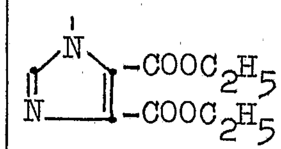 | very instable | very instable |
| comparison | $H_3CO$- 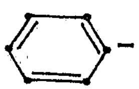 - | 2-$H_{33}C_{16}O$-;5-$H_3CO$- | theophyllinyl | stable | very instable |
| comparison | tert-butyl | 2-Cl;5-NHCO$(CH_2)_2$-O- 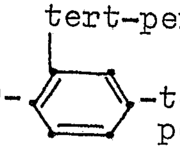 tert-pentyl, -tert-pentyl | 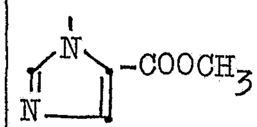 | stable | very instable |

| | | | | | |
|---|---|---|---|---|---|
| compound 1 | $H_{33}C_{16}O-$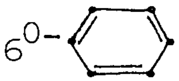- | 2,5-di-$H_3CO-$ | 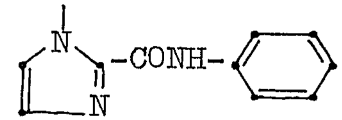 | stable | stable |
| compound 2 | $H_{33}C_{16}O-$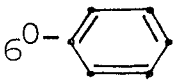- | 2,5-di-$H_3CO-$ | 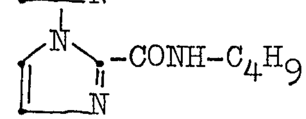 | stable | stable |
| compound 3 | $H_{33}C_{16}O-$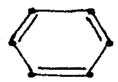- | 2,5-di-$H_3CO-$ | 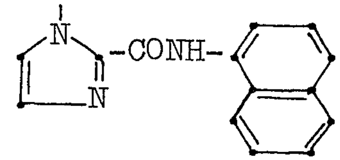 | stable | stable |
| compound 4 | $H_3CO-$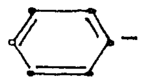- | 2-$H_{33}C_{16}O-$;5-$H_3CO$ | 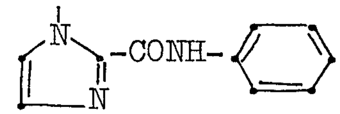 | stable | stable |
| compound 5 | $H_3CO-$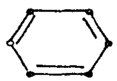- | 2-$H_{33}C_{16}O-$;5-$H_3CO$ | 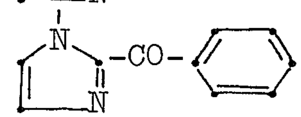 | stable | stable |
| compound 6 | tert-butyl | 2-Cl;5-NHCO$(CH_2)_3O-$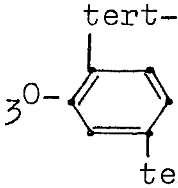 tert-pentyl, tert-pentyl | 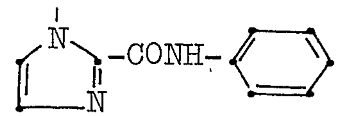 | stable | stable |
| compound 7 | tert-butyl | 2-Cl;5-NHCO$(CH_2)_3O-$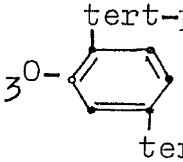 tert-pentyl, tert-pentyl | 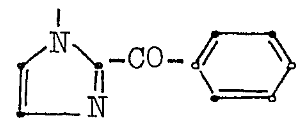 | stable | stable |

* The comparison couplers are couplers of the type described in the published German Patent Applications DE-OS 2,329,587 and DE-OS 2,559,190, both already mentioned hereinbefore.

The yellow-forming colour couplers according to the present invention are of the non-diffusing type since they comprise in their molecule an organic group sufficiently large to prevent the colour coupler from wandering from the colloid layer, in which the colour coupler has been incorporated, to another colloid layer.

For the preparation of a photographic multilayer colour element the non-diffusing colour couplers for each of the colour separation images are usually incorporated into the coating compositions of the differently sensitized silver halide emulsion layers. Yet, the non-diffusing colour couplers can also be added to the coating compositions of non-light-sensitive colloid layers that are in water-permeable relationship with the light-sensitive silver halide emulsion layers.

During the preparation of the light-sensitive colour element the non-migrating yellow-forming colour couplers according to the above general formula can be incorporated in the coating composition of the silver halide emulsion layers or other colloid layers in water-permeable relationship therewith according to any technique known by those skilled in the art for incorporating photographic ingredients, more particularly colour couplers, into colloid compositions.

The yellow-forming colour couplers according to the invention can be dispersed, occasionally in the presence of a wetting or dispersing agent, in a hydrophilic composition constituting or forming part of the binding agent of the colloid layer. Very suitable wetting agents that can be used to disperse the yellow-forming colour couplers of the invention are the fluorine-containing surface-active agents of the U.K. Patent Application n° 79/07040 filed on 28th February 1979 by Agfa-Gevaert N.V. For more details about particularly suitable techniques that can be employed for incorporating the colour

couplers of the invention into a hydrophilic colloid layer of a photographic element there can be referred to U.K. Patent Specifications 791,219 filed November 9, 1955 by Kodak Ltd., 1,098,594, 1,099,414, 1,099,415, 1,099,416, and 1,099,417, all filed January 25, 1965 by Gevaert-Agfa N.V., 1,199,570 filed October 31, 1966 by Agfa-Gevaert N.V., 1,218,190 filed October 2, 1968 by Gevaert-Agfa N.V., 1,297,947 filed March 20, 1969 by Agfa-Gevaert N.V., to the U.S. Patent Specifications 2,269,158 of Michele Martinez, issued January 6, 1942, 2,284,887 of Emil Vollenweider, issued June 2, 1942, 2,304,939 and 2,304,940, both of Leopold D.Mannes and Leopold Godowsky, Jr., issued December 15, 1942, and 2,322,027 of Edwin E.Jelley and Paul W.Vittum, issued June 15, 1943, and Belgian Patent Specification 722,026 filed October 9, 1968 by Gevaert-Agfa N.V.

Another technique for incorporating colour couplers is via polymeric latices as described in the published German Patent Applications DE-OS 2,541,230 and 2,541,274 both filed September 16, 1975 by Eastman Kodak Co.

The yellow-forming colour couplers according to the invention can be used in conjunction with various kinds of photographic emulsions. Various silver salts can be used as the light-sensitive salt. For instance silver bromide, silver iodide, silver chloride or mixed silver halides such as silver chlorobromide, silver bromoiodide, and silver chlorobromoiodide, can be employed. The couplers can be used in emulsions of the mixed packet type as decribed in the U.S. Patent Specification 2,698,794 of Leopold Godowsky, issued January 4, 1955 or emulsions of the mixed grain type as described in the U.S. Patent Specification 2,592,243 of Burt H. Carroll and Wesley T.Hanson, issued April 8, 1952. The colour couplers can be used with emulsions wherein

latent images are formed predominantly at the surface of the silver halide crystal or with emulsions wherein latent images are formed predominantly inside the silver halide crystal.

The hydrophilic colloid used as the vehicle for the silver halide can be e.g. gelatin, colloidal albumin, zein, casein, a cellulose derivative, a synthetic hydrophilic colloid such as polyvinyl alcohol or poly-N-vinyl pyrrolidone. If desired, compatible mixtures of two or more of these colloids can be employed for dispersing the silver halide.

The hydrophilic colloid may be replaced partly by polymers applied from aqueous dispersions known as latices. Particularly useful latices improving the properties such as flexibility of hydrophilic colloid layers are internally stabilized latices. These latices contain ionogenic monomer units in the polymer chain. Such latices are described e.g. in the United States Patent Specification 2,914,499 and in the published European Patent Application 0 010 335. In the latter Application copolymers are described containing ethyl acrylate units and monomers containing sulphonate groups e.g. monomers prepared by the condensation reaction of 11-methacryloylamino-undecanoyl chloride and taurine. Taurine may be advantageously replaced by an aromatic amino sulphonic acid resulting finally e.g. in a monomer having the following structure

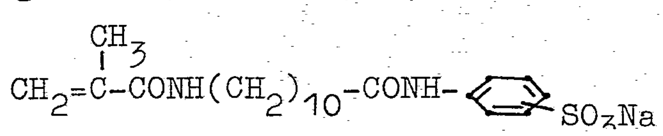

These ionogenic monomers may further be used advantageously for the preparation of copolymers including photographically useful groups as described e.g. in the European Patent Application 80 200 879 filed September 18, 1980 by Agfa-Gevaert N.V.

The light-sensitive silver halide emulsions used in the preparation of a photographic material according to the present invention can be sensitized chemically as well as optically. They can be sensitized chemically by carrying out the ripening in the presence of small amounts of sulphur-containing compounds such as allyl thiocyanate, allyl thiourea, or sodium thiosulphate. The emulsions can also be sensitized by means of reducing agents e.g. thin compounds as described in the French Patent Specification 1,146,955 filed April 11, 1956 by Gevaert Photo-Producten N.V. and in the Belgian Patent Specification 568,687 filed June 18, 1958 by Gevaert Photo-Producten N.V., imino-aminomethane sulphinic acid compounds as described in U.K. Patent Specification 789,823 filed April 29, 1955 by Gevaert Photo-Producten N.V., and small amounts of noble metal compounds such as gold, platinum, palladium, iridium, ruthenium, and rhodium compounds. They can be sensitized optically by means of cyanine and merocyanine dyes.

The said emulsions can also comprise compounds that sensitize the emulsions by development acceleration e.g. compounds of the polyoxyalkylene type such as alkylene oxide condensation products as described i.a. in U.S. Patent Specifications 2,531,832 of William Alexander Stanton, issued November 28, 1950, 2,533,990 of Ralph Kinsley Blake, issued December 12, 1950, in U.K. Patent Specifications 920,637 filed May 7, 1959, 940,051 filed November 1, 1961 and 945,340 filed October 23, 1961, all by Gevaert Photo-Producten N.V., 991,608 filed June 14, 1961 by Kodak Co. and 1,091,705 filed May 20, 1965 by Gevaert Photo-Producten N.V. and onium derivatives of amino-N-oxides as described in U.K. Patent Specification 1,121,696 filed October 7, 1965 by Gevaert-Agfa N.V.

Further, the emulsions may comprise stabilizers e.g. heterocyclic nitrogen-containing thioxo compounds such as benzothiazoline-2-thione and 1-phenyl-2-tetrazoline-5-thione and compounds of the hydroxytriazolopyrimidine type. They can also be stabilized with mercury compounds such as the mercury compounds described in Belgian Patent Specifications 524,121 filed November 7, 1953 by Kodak Co., 677,337 filed March 4, 1966 by Gevaert-Agfa N.V., and in the U.K. Patent Specification 1,173,609 filed December 12, 1966 by Agfa-Gevaert N.V.

The light-sensitive emulsion layers containing the colour couplers of the invention and adjacent hydrophilic colloid layers may comprise any other kind of ingredient such as those described for such emulsions and adjacent layers in Research Disclosure no. 17.643 of December 1978 e.g. plasticizers, hardening agents, wetting agents, etc.

The non-diffusing yellow-forming colour couplers of the present invention are usually incorporated into the blue-sensitive silver halide emulsion for forming one of the differently sensitized silver halide emulsion layers of a photographic multilayer colour element. Such photographic multilayer colour element usually comprises a support, a red-sensitized silver halide emulsion layer with a cyan-forming colour coupler, a green-sensitized silver halide emulsion layer with a magenta-forming colour coupler, and a blue-sensitive silver halide emulsion layer with a yellow-forming colour coupler. The emulsions can be coated on a wide variety of photographic emulsion supports. Typical supports include cellulose ester film, polyvinylacetal film, polystyrene film, polyethylene terephthalate film and related films or resinous materials, as well as paper and glass.

For the production of photographic colour images according to the present invention an exposed silver

halide emulsion layer is developed with an aromatic primary amino developing substance in the presence of a colour coupler according to the present invention. All colour developing agents capable of forming azomethine dyes can be utilized as developers. Suitable developing agents are aromatic compounds such as p-phenylene diamines such as N,N-diethyl-p-phenylene diamine, N,N-dialkyl-N'-sulphomethyl-p-phenylene diamines and N,N-dialkyl-N'-carboxymethyl-p-phenylene diamines.

The following example illustrates the present invention.

Example.

114.6 g of blue-sensitive silver bromoiodide emulsion (2.3 mole % of iodide) comprising per kg an amount of 73.4 g of gelatin and an amount of silver halide equivalent to 47 g of silver nitrate were diluted with 127 g of a 7.5% by volume solution of gelatin in 100 ml of distilled water.

A dispersion of yellow-forming colour coupler was made by dissolving 0.006 mole of the colour coupler as specified in the table hereinafter in 16 ml of ethyl acetate and 2 g of dibutyl phthalate, dispersing the resulting solution in 100 ml of a 5% by volume aqueous solution of gelatin containing 0.4 g of the sodium salt of dodecylbenzene sulphonic acid by means of an ultrasonic power generator, and eliminating the ethyl acetate by evaporation under reduced pressure.

The resulting dispersion was added to the blue-sensitive silver halide emulsion.

After neutralization of the emulsion and addition thereto of the usual additives such as stabilizing agents e.g. 5-methyl-7-hydroxy-s-triazolo[1,5-a]pyrimidine, wetting agents, and hardening agents the necessary amount of distilled water to obtain 575 g of emulsion was added.

The emulsion was coated on a film support in a ratio of 150 g per sq.m. The emulsion layer was dried and

covered with a gelatin layer. The dried emulsion material was cut and the resulting strips were exposed in a Herrnfeld sensitometer for 1/20th second through a continuous wedge with a constant of 0.30. After exposure the materials were colour processed in the usual way which includes colour development, bleaching, and fixing. Four developers were used.

- the first one containing as developing agent N,N-diethyl-p-phenylene diamine sulphate; known as TSS developing agent from Agfa-Gevaert A.G.; developing time : 8 minutes and the temperature of development : 20°C;
- the second one containing as developing agent 2-amino-5-diethylamino-toluene hydrochloride; known as CD-2 developing agent from Eastman Kodak; development time : 10 minutes; temperature of development : 24°C,
- the third one containing as developing agent 4-amino-N-ethyl-N-(β-methanesulphonamidoethyl)-m-toluidine sesquisulphate monohydrate known as CD-3 developing agent from Eastman Kodak; development time : 15 minutes; temperature of development : 21°C, and
- the fourth one containing as developing agent 4-amino-3-methyl-N-ethyl-N-(β-hydroxyethyl)aniline sulphate known as CD-4 developing agent from Eastman Kodak; development time : 10 minutes; temperature of development : 25°C.

In table 3 hereinafter the maximum densities of the yellow dye images obtained after development with the above-mentioned 4 developers of the strips of blue-sensitive emulsion containing the yellow-forming coupler compounds 1,3,4,5, and 9 are given.

TABLE 3 : Maximum density.

| Yellow-forming coupler in blue-sensitive emulsion | TSS-developer Dmax | CD-2 developer Dmax | CD-3 developer Dmax | CD-4 developer Dmax |
|---|---|---|---|---|
| compound 1 | 2.5 | 2.8 | 2.5 | 2.5 |
| 3 | 2.5 | 2.8 | 2.5 | 2.5 |
| 4 | 2.3 | 2.3 | 2.1 | 1.8 |
| 5 | 2.6 | 2.8 | 2.4 | 2.5 |
| 9 | 2.9 | 2.8 | 2.8 | 2.6 |

WE CLAIM :

1. Colour coupler of the acylacetanilide type, capable of forming a yellow azomethine dye by reaction with an oxidized aromatic primary amino developing agent, said colour coupler carrying on the methylene group between the two carbonyl groups a 1-imidazolyl coupling-off group, characterized in that said 1-imidazolyl group is a 2-arylcarbamoyl-imidazol-1-yl group, a 2-alkylcarbamoyl-imidazol-1-yl group, or a 2-aroyl-imidazol-1-yl group.

2. A colour coupler according to claim 1, characterized in that it corresponds to the following general formula :

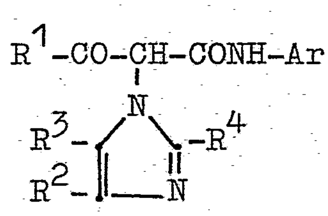

wherein :

Ar represents phenyl or phenyl substituted by 1 to 3 substituents, which may be the same or different and are selected from the group consisting of a branched or unbranched alkyl group having 1 to 18 carbon atoms, an alkoxy group having 1 to 18 carbon atoms, a halogen atom, acylamido or substituted acylamido, carbamoyl or substituted carbamoyl, sulphamoyl or substituted sulphamoyl, carboxy, nitro, alkylsulphonyl, or arylsulphonyl,

$R^1$ represents a branched or unbranched alkyl group having 1 to 18 carbon atoms, an alkoxy group having 1 to 18 carbon atoms, an alkoxyalkyl group, a cycloalkyl group, a heterocyclic group, an aryl group or a substituted aryl group,

$R^2$ and $R^3$ are substituents used in colour coupler chemistry, which may be the same or different, these substituents being hydrogen, alkyl, alkoxy, alkylthio, aryl or substituted aryl, acylamido or substituted acylamido, carbamoyl or substituted carbamoyl, sulphamoyl or substituted sulphamoyl, sulphoamino, alkoxycarbonyl, carboxy, a halogen atom, or cyano; or $R^2$ and $R^3$ together represent the atoms necessary to complete a 5- or 6-membered unsaturated carbocyclic ring, which may be substituted;

$R^4$ represents an aroyl group, an arylcarbamoyl group, or an alkylcarbamoyl group.

3. A colour coupler according to claim 2, characterized in that $R^1$ is tert.butyl, phenyl or substituted phenyl and each of $R^2$ and $R^3$ is hydrogen.

4. Photographic element comprising a light-sensitive silver halide emulsion layer and at least one colour coupler according to any of claims 1 to 3.

5. A photographic element according to claim 4, characterized in that said element is a photographic multilayer colour element comprising three silver halide emulsion layers, which are differently optically sensitized and said colour coupler(s) is (are) present in a blue-sensitive silver halide emulsion layer or a non-light-sensitive colloid layer in water-permeable relationship therewith.

6. Process for the production of a photographic colour image by development of a photographic element containing image-wise exposed silver halide with the aid of a developing agent, which by reduction of the exposed silver halide is converted into its oxidized form and as such forms a yellow azomethine dye by reaction with a colour coupler according to any of claims 1 to 3.

7. Process according to claim 6, characterized in that said colour coupler is present in a blue-sensitive

silver halide emulsion layer or other colloid layer in water-permeable relationship therewith of the said photographic element.

0030747

European Patent Office

# EUROPEAN SEARCH REPORT

Application number
EP 80 20 1054

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| X | US - A - 4 049 458 (I. BOIE et al.)<br>* Column 2, line 1 - column 4, line 28; column 19, lines 37-51; claims 1,2 *<br>-- | 1-7 | G 03 C 7/36 |
| | DE - A - 2 536 191 (FUJI PHOTO)<br>* Page 15, line 13 - page 20, line 5; claim 1 *<br>& GB - A - 1 516 547<br>& US - A - 4 241 168<br>---- | 1 | |

TECHNICAL FIELDS SEARCHED (Int. Cl.3)

G 03 C 7/36
7/34
7/32
7/38

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 13-03-1981 | PHILOSOPH |